# FASCICULE DE BREVET EUROPEEN

(11) **EP 2 488 050 B1**
(45) Date de publication et mention de la délivrance du brevet: **11.09.2013**
(21) Numéro de dépôt: 10763723.3
(22) Date de dépôt: 14.10.2010
(51) Int. Cl.: A23K 1/16, C12P 13/12

(54) **COMPOSITION RICHE EN METHIONINE DESTINEE A L'ALIMENTATION ANIMALE**
ZUSAMMENSETZUNG MIT HOHEM METHIONINGEHALT ZUR FÜTTERUNG VON TIEREN
METHIONINE-RICH COMPOSITION FOR FEEDING ANIMALS

(30) Priorité: 14.10.2009 FR 0957183
(43) Date de publication de la demande: 22.08.2012
(73) Titulaire: Roquette Freres, 62136 Lestrem (FR)
(72) Inventeur: FUERTES, Patrick, 59160 Lomme (FR)
(74) Mandataire: Gallois, Valérie
(86) Numéro de dépôt international: PCT/EP2010/065428
(87) Numéro de publication internationale: WO 2011/045377

(56) Documents cités:
- EP-A1- 0 588 707
- EP-A1- 0 992 490
- WO-A2-2007/135188
- WO-A2-2009/043803
- GB-A- 1 296 347
- US-B1- 6 417 395

## Description

La présente invention concerne une nouvelle composition de méthionine, liquide ou cristalline, obtenue par biotransformation de biomasse par des microorganismes appropriés pour la production de méthionine.

La méthionine, comme d'autres acides aminés soufrés, est indispensable au métabolisme cellulaire. La méthionine n'est toutefois pas produite par les animaux qui doivent donc en trouver en quantités suffisantes dans leur alimentation. Elle est donc produite de manière industrielle pour être ajoutée comme compléments alimentaires, en particulier pour l'alimentation animale. La méthionine peut aussi être employée comme médicament dans le traitement ou la prévention de maladies diverses comme les allergies ou les fièvres rhumatoïdes.

Les sources usuelles de méthionine sont soit les protéines d'origine animale, soit la synthèse chimique. Toutefois, la diminution de l'utilisation des protéines animales suite au développement de l'ESB ou de la grippe aviaire à conduit en une augmentation de la demande en méthionine de synthèse.

La D,L-méthionine est généralement produite à partir de ressources fossiles et de dérivés de la pétrochimie, en particulier à partir d'acroléine, de méthyl mercaptan et de cynaures. L'obtention de l'énantiomère L plus actif, nécessite des étapes supplémentaires de résolution de racémique qui en augmentent drastiquement les coûts de production.

Aujourd'hui, la production de méthionine par biotransformation constitue une alternative avantageuse à la pétrochimie du fait de la raréfaction des ressources fossiles et de l'augmentation du coût des matières premières. La mise en oeuvre de ces procédés nécessite toutefois de disposer de microorganismes appropriés pour produire de la méthionine par biofermentation sur une source de carbone.

Les premières solutions efficaces industriellement ont été publiées, et notamment décrites dans les demandes de brevet WO 2005/111202, WO 2007/017710, WO 2007/077041, WO 2007/135188 et WO 2009/043803. D'autres microorganismes produisant de la méthionine sont également décrits dans les demandes de brevet WO 2004/038 013, WO 2006/001616, WO 2006/138689 et WO 2007/012078, notamment.

La production à grande échelle de méthionine biosynthétique rencontre toutefois des problèmes propres à la récupération de molécules chimiques dans un fermenteur, notamment pour la purification des produits finis. Dans ce cas, la qualité du mélange brut obtenu, la teneur en impuretés et leur nature revêt une grande importance.

La présente invention concerne donc une nouvelle composition liquide de méthionine, issue d'un procédé de fermentation, dont la teneur en méthionine et en autres constituants permet une purification plus aisée, en particulier pour l'obtention d'un produit solide comprenant une teneur supérieure en méthionine par rapport aux résidus secs de la composition liquide.

La composition de méthionine est issue d'un procédé de fermentation, c'est-à-dire de transformation de biomasse, dans lequel on cultive un microorganisme modifié pour produire de la méthionine sur un milieu de culture approprié comprenant une source de carbone. Les sources de carbone sont choisies parmi toutes les sources de carbone susceptibles d'être métabolisées par un microorganisme, et en particulier le glucose, le sucrose, les mono- ou oligosaccharides, l'amidon et ses dérivés et leurs mélanges.

Dès lors, la composition selon l'invention se distingue de compositions de méthionine obtenues par d'autres procédés, notamment chimiques, par la teneur en isotopes du carbone des molécules de méthionine qui sont spécifiques de produits de fermentation par opposition des produits issus de matières premières fossiles. La composition selon l'invention peut aussi se distinguer des compositions de méthionine obtenues par d'autres procédés par la nature et/ou la teneur des impuretés présentes.

La composition selon l'invention est une composition de méthionine issue d'un procédé de fermentation comprenant de 50 à 95% en poids de méthionine et comprenant moins de 10% en poids d'isoleucine. De manière préférentielle, la composition comprend de 60 à 95% en poids de méthionine.

Sauf indication contraire, les pourcentages sont donnés ici en poids des résidus secs de la composition, qu'il s'agisse d'une composition liquide ou solide.

Plus particulièrement, la composition selon l'invention est un additif alimentaire comprenant, voire consistant en, une composition de méthionine issue d'un procédé de fermentation, notamment tel que défini dans la présente description.

Selon un de ses aspects, la présente invention a pour objet un additif alimentaire pour l'alimentation animale comprenant, voire consistant en, une composition de méthionine issue d'un procédé de fermentation comprenant :
- de 60 à 95%, notamment de 70 à 95%, en poids de méthionine,
- de 0,05 à 2,5%, notamment de 0,1 à 2%, en particulier de 0,3 à 2% en poids de N-acétyl méthionine, et
- de 0,05 à 3,5%, notamment de 0,2 à 3%, en particulier de 0,3 à 3% en poids d'isoleucine.

Tout particulièrement, l'additif alimentaire peut comprendre, voire consister, en une composition comprenant en outre :
- de 1,0 à 6,0% en poids, notamment de 2,0 à 5,0% en poids, d'acides aminés autres que méthionine, N-acétyl méthionine et isoleucine
- de 0,1 à 2% en poids, notamment de 0,2 à 1,5% en poids, de sucres totaux et
- de 0,05 à 2,5% en poids, notamment de 0,1 à 2% en poids, en particulier de 0,2 à 1,5% en poids de lipides.

La composition ou l'additif selon l'invention peut être une composition liquide, aqueuse, et dans ce cas il s'agit plus particulièrement de la composition obtenue en fin de fermentation, après élimination de la biomasse et d'une partie des impuretés organiques. On la désignera comme une composition « brute ».

Selon une variante, lorsque la composition, ou l'additif, est liquide, elle ou il se présente sous forme de solution, en particulier claire. Cette composition, ou additif, sous forme liquide peut être dépourvu(e) de méthionine sous forme solide et en particulier cristallisée.

La composition, ou l'additif, sous forme liquide peut par ailleurs se présenter sous forme de dispersion.

Tout particulièrement, la teneur en méthionine de cette composition ou additif sous forme liquide est inférieure ou égale à 8 g/100 ml, notamment à 4,8 g/100 ml, en particulier à 4,5 g/100 ml, voire à 4 g/100 ml de composition liquide.

De manière avantageuse, la composition, ou l'additif, liquide selon l'invention comprend entre 60 et 85% en poids de méthionine, plus particulièrement entre 70 et 85%.

La composition ou l'additif liquide peut comprendre une teneur en méthionine allant de 60 à 80% en poids, notamment de 65 à 75% en poids.

La composition ou l'additif liquide peut comprendre une teneur en N-acétyl méthionine allant de 0,5 à 2,0% en poids, notamment de 0,75 à 1,75% en poids.

La composition ou l'additif liquide peut comprendre une teneur en isoleucine allant de 0,20 à 1,5% en poids, notamment de 0,3 à 1,0%, en poids.

La composition ou l'additif liquide peut comprendre une teneur en acides aminés autres que méthionine, N-acétyl méthionine et isoleucine allant de 2 à 6% en poids, notamment de 3 à 5% en poids.

La composition ou l'additif liquide peut comprendre une teneur en sucres totaux allant de 0,5 à 2% en poids, notamment de 0,75 à 1,5% en poids.

La composition ou l'additif liquide peut comprendre une teneur en lipides allant de 0,05 à 2,5% en poids, notamment de 0,075 à 1,5% en poids.

Ainsi, selon un premier mode de réalisation, l'additif alimentaire sous forme liquide comprend, voire consiste en, une composition comprenant :
- de 60 à 80% en poids, notamment de 65 à 75% en poids, de méthionine,
- de 0,5 à 2,0% en poids, notamment de 0,75 à 1,75% en poids, de N-acétyl méthionine, et
- de 0,20 à 1,5% en poids, notamment de 0,3 à 1,0%, en poids, d'isoleucine.

Encore plus particulièrement, l'additif alimentaire sous forme liquide comprend, voire consiste en, une composition comprenant :
- de 2 à 6% en poids, notamment de 3 à 5% en poids, d'acides aminés autres que méthionine, N-acétyl méthionine et isoleucine,
- de 0,5 à 2% en poids, notamment de 0,75 à 1,5% en poids, de sucres totaux et
- de 0,05 à 2,5% en poids, notamment de 0,075 à 1,5% en poids, de lipides.

La composition ou l'additif liquide peut encore comprendre une teneur en méthionine allant 70 à 90% en poids, notamment de 75 à 85% en poids.

La composition ou l'additif liquide peut comprendre une teneur en N-acétyl méthionine allant de 0,05 à 2,5% en poids, notamment de 0,075 à 2,0% en poids.

La composition ou l'additif liquide peut comprendre une teneur en isoleucine allant de 0,1 à 1,0% en poids, notamment de 0,3 à 0,75% en poids.

La composition ou l'additif liquide peut comprendre une teneur en acides aminés autres que méthionine, N-acétyl méthionine et isoleucine allant de 2 à 6% en poids, notamment de 3 à 5% en poids.

La composition ou l'additif liquide peut comprendre une teneur en sucres totaux allant de 0,25 à 2% en poids, notamment de 0,5 à 1,5% en poids.

La composition ou l'additif liquide peut comprendre une teneur en lipides allant de 0,05 à 2,5% en poids, notamment de 0,075 à 2,0% en poids.

Ainsi, selon un deuxième mode de réalisation, l'additif alimentaire sous forme liquide comprend, voire consiste en, une composition comprenant :
- de 70 à 90% en poids, notamment de 75 à 85% en poids, de méthionine,
- de 0,05 à 2,5% en poids, notamment de 0,075 à 2,0% en poids, de N-acétyl méthionine, et
- de 0,1 à 1,0% en poids, notamment de 0,3 à 0,75%, en poids d'isoleucine.

Encore plus particulièrement, l'additif alimentaire sous forme liquide comprend, voire consiste en, une composition comprenant :
- de 2 à 6% en poids, notamment de 3 à 5% en poids, d'acides aminés autres que méthionine, N-acétyl méthionine et isoleucine
- de 0,25 à 2% en poids, notamment de 0,5 à 1,5% en poids, de sucres totaux et
- de 0,05 à 2,5% en poids, notamment de 0,05 à 2,0% en poids, de lipides.

La composition selon l'invention peut se présenter sous forme solide. Cette composition est plus particulièrement le produit de cristallisation de la méthionine contenue dans la composition liquide définie précédemment après un traitement de purification.

De manière avantageuse, la composition solide selon l'invention comprend entre 75 et 95% en poids de méthionine, plus particulièrement entre 85 et 95%.

Par ailleurs, l'additif alimentaire peut se présenter sous forme solide.

La composition ou l'additif solide peut comprendre une teneur en méthionine allant de 80 à 95% en poids, notamment de 85 à 95% en poids.

La composition ou l'additif solide peut comprendre une teneur en N-acétyl méthionine allant de 0,05 à 0,5% en poids, notamment de 0,05 à 0,3% en poids.

La composition ou l'additif solide peut comprendre une teneur en isoleucine allant de 0,05 à 5,0% en poids, notamment de 0,1 à 3,0% en poids.

La composition ou l'additif solide peut comprendre une teneur en acides aminés autres que méthionine, N-acétyl méthionine et isoleucine allant de 1,0 à 5,0% en poids, notamment de 1,5 à 4,0% en poids.

La composition ou l'additif solide peut comprendre une teneur en sucres totaux allant de 0,1 à 1,5% en poids, notamment de 0,2 à 1,0% en poids.

La composition ou l'additif solide peut comprendre une teneur en lipides inférieure ou égale à 0,3% en poids.

Selon un troisième mode de réalisation, l'additif alimentaire, notamment solide, comprend, voire consiste en, une composition comprenant :
- de 80 à 95% en poids, notamment de 85 à 95% en poids, de méthionine,
- de 0,05 à 0,5% en poids, notamment de 0,05 à 0,3% en poids, de N-acétyl méthionine, et
- de 0,1 à 5,0% en poids, notamment de 0,1 à 3,0% en poids, d'isoleucine.

Encore plus particulièrement, l'additif alimentaire sous forme liquide comprend, voire consiste en, une composition comprenant :
- de 1,0 à 5,0% en poids, notamment de 1,5 à 4,0% en poids, d'acides aminés autres que méthionine, N-acétyl méthionine et isoleucine
- de 0,1 à 1,5% en poids, notamment de 0,2 à 1,0% en poids, de sucres totaux et
- moins de 0,3% en poids de lipides.

La composition selon l'invention, issue d'un procédé de fermentation, comprend avantageusement moins de 10% en poids d'isoleucine plus particulièrement moins de 5% en poids.

La composition selon l'invention est une composition qui comprend généralement d'autres résidus issus du procédé de bioproduction de méthionine et en particulier d'autres acides aminés. La teneur en acides aminés autres que la méthionine et l'isoleucine, est avantageusement inférieure à 10% en poids. De nombreux acides aminés sont présents comme résidus dans la composition selon l'invention et la teneur de chaque acide aminé autre que la méthionine et l'isoleucine pris individuellement est avantageusement inférieure à 2% en poids, plus avantageusement inférieure à 1 % en poids.

La composition selon l'invention comprend avantageusement moins de 0,2% de cendres, et en particulier de 0,01 à 0,2% de cendres.

La teneur en N-acétyl-méthionine est avantageusement comprise entre 0 et 2% en poids. Elle dépendra notamment du caractère liquide ou solide de la composition brute. Sa teneur se rapproche de 0 dans la composition solide.

La composition selon l'invention comprend également et avantageusement moins de 2% en poids de lipides et moins de 2% en poids de sucres, avantageusement moins de 1% en poids.

Le procédé d'obtention des compositions est constitué de plusieurs étapes :
A) clarifier le milieu de fermentation et éliminer les impuretés organiques insolubles et solubles dudit milieu de fermentation pour l'obtention d'une composition brute liquide,
   le cas échéant
B) déminéraliser la solution brute liquide afin d'éliminer les cations et anions dudit milieu de fermentation, pour l'obtention d'une composition brute liquide déminéralisée, et garantir l'obtention d'une méthionine solide au profil hygroscopique proche de celui de la méthionine chimique,
   le cas échéant
C) cristalliser la méthionine brute à partir de la solution liquide déminéralisée, et
D) séparation, éventuellement lavage, et séchage et éventuellement broyage des cristaux de méthionine pour l'obtention d'une composition brute solide.

Il est entendu qu'une composition comprenant de la méthionine cristallisée dans une solution de méthionine fait également partie de l'invention.

On entend au sens de l'invention par « impuretés organiques insolubles » la biomasse, les protéines et les particules insolubles résiduelles.

Par « impuretés organiques solubles », on entend toutes les particules solubles contaminant le milieu de fermentation, notamment les macromolécules du type protéines solubles et des polysaccharides.

La première étape du procédé consiste à clarifier le milieu de fermentation et à éliminer les impuretés organiques. La clarification du milieu est réalisée par toute méthode connue en tant que telle par l'Homme du métier, méthode choisie par exemple dans le groupe constitué de la floculation, de la décantation, des techniques membranaires (microfiltration, ultrafiltration, nanofiltration et osmose inverse) et de la centrifugation. L'élimination des impuretés organiques solubles est réalisée par toute méthode connue en tant que telle par l'Homme du métier, méthode choisie par exemple dans le groupe constitué de l'ultrafiltration, du traitement thermique, du traitement à l'aide d'un adsorbant de type charbon actif et de l'hydrolyse enzymatique. L'élimination de ces impuretés solubles permet de garantir le bon comportement de la masse cuite lors de la cristallisation. En effet, sans cette étape, la masse cuite a un aspect homogène pâteux, générant des cristaux très fins et pénalisant leur séparation ainsi que leur lavage. En conséquence, la pureté des cristaux est diminuée.

La deuxième étape consiste à éliminer les sels minéraux de la solution ainsi obtenue. Cette étape peut être réalisée par électrodialyse conventionnelle (EURODIA^{®}) et/ou par traitement sur résine échangeuse de cations sous forme H⁺ (PUROLITE^{®} C120, PUROLITE^{®} C150, PUROLITE^{®} C160...) et/ou résine échangeuse d'anions (LEWATIT^{®} S4228, LEWATIT^{®} S4528, Rohm & Haas FPA91...). Le traitement par résines échangeuses d'ions sera préféré à l'EDC pour des raisons de coût et d'efficacité d'abattement des sels. Cette étape permet entre autres de réduire la teneur en sels dans la méthionine solide et ainsi d'assurer une reprise en eau minimale pour une bonne stabilité au stockage.

La troisième étape consiste à cristalliser la méthionine de manière à récupérer la méthionine brute sous forme solide. Cette étape de cristallisation peut être réalisée par une technologie choisie dans le groupe constitué de la cristallisation par refroidissement, de la cristallisation par évapocristallisation et de la cristallisation adiabatique. La société demanderesse recommande d'utiliser l'évapocristallisation. Si l'évapocristallisation est choisie, la société Demanderesse recommande de pré-concentrer la solution de méthionine brute par évaporation sous vide à l'aide d'un évaporateur à film tombant afin de s'approcher de la sursaturation. La solution pré-concentrée est alors transférée dans un cristallisoir de type Draft tube, par exemple, pour y être concentrée davantage et cristalliser. La solubilité de la méthionine à 35°C est d'environ 70g/L. En concentrant la solution à environ 250g/L, sous un vide assurant une température de 35°C, le rendement de récupération en méthionine est > 70%. Dans ces conditions, et grâce à l'élimination des impuretés solubles, la méthionine solide cristallise sous forme de sphères spongieuses, faciles à séparer des eaux mères.

La quatrième étape consiste à séparer la méthionine brute solide ainsi obtenue, à la laver et à la sécher. La récupération de la méthionine brute solide est réalisée par toute méthode connue en tant que telle par l'Homme du métier, méthode choisie par exemple dans le groupe constitué de la centrifugation, l'essorage et de la filtration frontale (sur tambour, sur filtre presse, ...). Dans ce dernier cas, la méthionine brute est alors retenue sur une toile de 40µm et les eaux-mères passent à travers. Le gâteau de méthionine brute est ensuite lavé avec 1 à 10BV d'eau de préférence déminéralisée. La méthionine brute solide est ensuite séchée et éventuellement broyée pour classifier les particules.

La composition brute, liquide et solide, obtenue est décrite dans le Tableau 1 ci-dessous :

**Tableau 1**

| **G / 100g de résidu sec** | **Composition brute liquide** | **Composition brute solide** |
|---|---|---|
| L-Méthionine (L-MET) | 50 - 85 | 75 - 95 |
| N-acétyl Méthionine (NAM) | < 2 | < 1 |
| Isoleucine (ISO) | < 10 | < 10 |
| Dérivés d'AA inconnu | < 2 | < 2 |
| Valine | < 2 | < 2 |
| Phénylalanine | < 2 | < 2 |
| Leucine | < 2 | < 2 |
| Acide aspartique | < 2 | < 2 |
| Thréonine | < 2 | < 2 |
| Alanine | < 2 | < 2 |
| Lysine | < 2 | < 2 |
| Arginine | < 2 | < 2 |
| Cystine | < 2 | < 2 |
| Glutamine | < 2 | < 2 |
| Tyrosine | < 2 | < 2 |
| Acide glutamique | < 2 | < 2 |
| Glycine | < 2 | < 2 |
| Citrulline | < 2 | < 2 |
| Total AA hors L-MET, NAM et ISO | < 10 | < 10 |
| Sucres totaux | < 5 | < 2 |
| Lipides | < 2 | < 2 |
| Acides organiques | < 3 | < 2 |
| Thiosulfates | <7 | < 1 |
| Sulfates | < 1,5 | < 2 |
| Phosphates | < 0,2 | < 1 |
| Chlorures | < 0,2 | < 0,5 |
| Ammonium | < 3 | < 1,5 |
| Sodium | < 1 | < 0,01 |
| Potassium | < 1 | < 0,05 |
| Magnésium | < 0,2 | < 0,01 |
| Calcium | < 0,1 | < 0,01 |
| Autres | < 10 | < 5 |

La ligne « Dérivés d'AA inconnu » correspond à des espèces proches des AA et en cours d'identification.

La ligne « Autres » correspond à des espèces non identifiées à ce jour.

La composition brute selon l'invention peut avantageusement être employée directement, sous forme solide ou liquide, dans l'alimentation animale comme complément ou additif alimentaire fourni aux animaux, mélangé au bol alimentaire fourni à chaque animal, en prémélange, sous la forme d'une composition prémélangée ou de manière extemporanée, ou de manière indépendante des autres aliments.

L'invention concerne donc également un additif alimentaire comprenant la composition de méthionine selon l'invention.

Selon encore un autre de ses aspects, l'invention a encore pour objet un procédé de préparation d'un additif alimentaire pour l'alimentation animale, notamment tel que défini ci-dessus, comprenant, voire consistant en, au moins l'étape suivante consistant, à partir d'un milieu de fermentation d'un procédé de préparation de méthionine dans lequel on cultive un microorganisme modifié pour produire de la méthionine sur un milieu de culture approprié comprenant une source de carbone, à :
A) clarifier le milieu de fermentation et éliminer les impuretés organiques insolubles et solubles dudit milieu de fermentation pour l'obtention d'une composition brute liquide comprenant :
   - de 60 à 80% en poids, notamment de 65 à 75% en poids, de méthionine,
   - de 0,5 à 2,0% en poids, notamment de 0,75 à 1,75% en poids, de N-acétyl méthionine, et
   - de 0,20 à 1,5% en poids, notamment de 0,3 à 1,0%, en poids, d'isoleucine, et
E) récupérer l'additif alimentaire.

Le procédé de préparation peut conduire à une composition brute liquide comprenant en outre :
- de 2 à 6% en poids, notamment de 3 à 5% en poids, d'acides aminés autres que méthionine, N-acétyl méthionine et isoleucine
- de 0,5 à 2% en poids, notamment de 0,75 à 1,5% en poids, de sucres totaux et
- de 0,05 à 2,5% en poids, notamment de 0,075 à 1,5% en poids, de lipides.

Le procédé de préparation d'un additif alimentaire peut comprendre en outre une étape consistant à :
B) déminéraliser la solution brute liquide afin d'éliminer les cations et anions dudit milieu de fermentation, notamment pour l'obtention d'une composition brute liquide déminéralisée comprenant :
   - de 70 à 90% en poids, notamment de 75 à 85% en poids, de méthionine,
   - de 0,05 à 2,5% en poids, notamment de 0,075 à 2,0% en poids, de N-acétyl méthionine, et
   - de 0,1 à 1,0% en poids, notamment de 0,3 à 0,75%, en poids d'isoleucine.

Le procédé de préparation d'un additif alimentaire peut permettre l'obtention d'une composition brute liquide déminéralisée comprenant en outre :
- de 2 à 6% en poids, notamment de 3 à 5% en poids, d'acides aminés autres que méthionine, N-acétyl méthionine et isoleucine
- de 0,25 à 2% en poids, notamment de 0,5 à 1,5% en poids, de sucres totaux et
- de 0,05 à 2,5% en poids, notamment de 0,075 à 2,0% en poids, de lipides.

Plus particulièrement, le procédé de préparation d'un additif alimentaire permet d'obtenir un additif alimentaire liquide, et notamment ledit procédé ne comprend pas d'étape de cristallisation.

Le procédé de préparation d'un additif alimentaire peut comprendre en outre l'étape consistant à :
C) cristalliser de la méthionine brute solide à partir de la composition brute liquide, éventuellement déminéralisée.

Le procédé de préparation d'un additif alimentaire peut encore comprendre l'étape consistant à :
D) séparer, éventuellement laver, et éventuellement broyer des cristaux de méthionine pour l'obtention d'une composition brute solide.

Enfin, le procédé de préparation d'un additif alimentaire peut permettre d'obtenir une composition brute solide comprenant :
- de 80 à 95% en poids, notamment de 85 à 95% en poids, de méthionine,
- de 0,05 à 0,5% en poids, notamment de 0,05 à 0,3% en poids, de N-acétyl méthionine, et
- de 0,05 à 5,0% en poids, notamment de 0,1 à 3,0%, en poids d'isoleucine.

Le procédé de préparation d'un additif alimentaire peut permettre que la composition méthionine brute solide ou la composition brute solide comprenne en outre :
- de 1,0 à 5,0% en poids, notamment de 1,5 à 4,0% en poids, d'acides aminés autres que méthionine, N-acétyl méthionine et isoleucine,
- de 0,1 à 1,5% en poids, notamment de 0,2 à 1,0% en poids, de sucres totaux et
- moins de 0,3% en poids de lipides.

Par ailleurs, l'invention a également pour objet un procédé de préparation d'un aliment complémenté en méthionine comprenant au mois une étape d'addition d'une composition ou d'un additif alimentaire selon l'invention.

Selon une variante cette addition est effectuée par addition d'une composition ou d'un additif sous forme liquide sur un aliment solide. Ceci peut en particulier être effectué de manière extemporanée. L'addition peut ainsi être effectuée par une simple pulvérisation de la composition ou de l'additif.

Selon une autre variante cette addition est effectuée par addition d'une composition ou d'un additif sous forme liquide à un aliment liquide, comme de l'eau de boisson. Ceci peut également être effectué de manière extemporanée.

Selon encore un autre de ses aspects, l'invention a pour objet l'utilisation d'une composition telle que définie ci-dessus pour la préparation d'un additif alimentaire et/ou d'un aliment complémenté en méthionine.

L'homme du métier connait bien les quantités en méthionine nécessaires à l'alimentation animale dans un régime alimentaire approprié à chaque animale et saura déterminer comment employer la composition selon l'invention et en quelle quantité. En particulier, la forme brute liquide est particulièrement adaptée pour son apport en oligoéléments et en eau pour faciliter le dosage, le mélange et l'hydratation des aliments habituels de l'animal.

La composition de méthionine brute selon l'invention peut être issue de tout procédé de bioproduction de méthionine avec culture d'un microorganisme optimisé pour favoriser la synthèse de méthionine, qu'il s'agisse d'une bactérie, de levures ou de champignons (moisissures). Avantageusement, le microorganisme est choisi parmi les *Enterobacteriaceae, Bacillaceae, Streptomycetaceae* et *Colynebacteriaceae.* Plus particulièrement, le microorganisme est une espèce choisie parmi les espèces *Escherichia, Klebsiella, Pantoea, Salmonella* ou *Corynebacterium.* Plus particulièrement, le microorganisme est choisi parmi les espèces *Escherichia coli* ou *Corynebacterium glutamicum.*

Dans un mode particulier de réalisation de l'invention, la composition de méthionine brute selon l'invention provient de la culture des microorganismes décrits dans la demande internationale WO 2009/043803 dont le contenu est incorporé ici par référence, et plus particuliers les microorganismes décrits dans les exemples de réalisation.

L'invention est plus particulièrement illustrée dans l'exemple de réalisation exposé ci-après.

Une souche de E. coli productrice de méthionine de génotype MG 1655 metA* 11 Ptrc-metH PtrcF-cysPUWAM PtrcF-cysJIH Ptrc09-gcvTHP Ptrc36-ARNmst17-metF ΔmetJ ΔpykF ΔpykA ΔpurU (pME 101-thrA*1-cysE-PgapA-metA*11) (pCC1BAC-serB-serA-serC), décrite dans la demande internationale WO 2009/043803, est cultivée dans des conditions de culture de fermentation selon la méthode décrite dans cette même demande internationale.

### Exemple 1 : Préparation de Méthionine Brute

Un moût de fermentation issu de la mise en oeuvre de la souche précédemment décrite et contenant de la L-méthionine est purifié comme suit.

### A) Elimination des impuretés organiques insolubles : la biomasse

L'élimination est réalisée par filtration tangentielle sur membrane présentant un diamètre de pore de 100 nm, entre 40 et 80°C (membrane de type céramique de 3,5 mm de diamètre de canal).

La température est maintenue préférentiellement à 40°C avec une pression transmembranaire de 1 bar et une diafiltration avec 20% d'eau déminéralisée.

Dans ces conditions, le flux moyen est de 30 L/h/m² et le perméat obtenu est limpide et brillant. Le perméat, débarrassé de la biomasse et des particules insolubles, contient encore des impuretés organiques solubles, notamment des sucres et protéines solubles qu'il convient d'éliminer avant cristallisation.

### B) Elimination des impuretés organiques solubles : les sucres et macromolécules solubles

Cette étape a pour objectif d'éliminer les sucres (polysaccharides) et les macromolécules contenues dans le moût de fermentation. En effet, ces impuretés affectent négativement le comportement de la masse cuite (aspect lié, homogène, cristaux très fins) et la récupération de la phase solide lors de l'étape de cristallisation de la L-méthionine.

Cette élimination peut être réalisée par ultrafiltration sur membrane céramique présentant un seuil de coupure de 5kDa. A 40°C, le flux de filtration est en moyenne de 25L/h/m² et environ 70% des macromolécules sont retenus dans le rétentat.

Le perméat, correspondant à la composition brute liquide, a la composition suivante :

**Tableau 2**

| **g / 100g de résidu sec** | **Composition brute liquide** |
|---|---|
| L-Méthionine (L-MET) | 72,6 |
| N-acétyl Méthionine (NAM) | 1,36 |
| Isoleucine (ISO) | 0,50 |
| Dérivé d'AA inconnu | 1,2 |
| Valine | 0,61 |
| Phénylalanine | 0,58 |
| Leucine | 0,18 |
| Acide aspartique | 0,25 |
| Thréonine | 0,26 |
| Alanine | 0,39 |
| Lysine | 0,10 |
| Arginine | 0,04 |
| Cystine | 0,02 |
| Glutamine | 0,17 |
| Tyrosine | 0,28 |
| Acide glutamique | 0,17 |
| Glycine | 0,10 |
| Citrulline | 0,15 |
| AA hors MET, NAM et ISO | 3,99 |
| Sucres totaux | 0,89 |
| Lipides | 1,05 |
| Acides organiques | < 3 |
| Thiosulfates | 4,72 |
| Sulfates | 1,04 |
| Phosphates | 0,09 |
| Chlorures | 0,10 |
| Ammonium | 2,26 |
| Sodium | 0,58 |
| Potassium | 0,42 |
| Magnésium | 0,10 |
| Calcium | 0,02 |
| Autres | 8,47 |

Ce perméat est alors déminéralisé avant d'être cristallisé pour conduire à la composition brute solide.

### C) Déminéralisation du perméat :

Cette étape de déminéralisation a pour objectif d'éliminer les anions et les cations minéraux présents dans le moût de fermentation contenant la L-méthionine.

Elle peut être conduite par électrodialyse conventionnelle et/ou traitement sur résine échangeuse de cation fort et sur résine échangeuse d'anion.

Lorsque le traitement est réalisé sur résines échangeuses d'ions, le pourcentage de déminéralisation est supérieur à celui atteint en électrodialyse conventionnelle. Ainsi avec une résine échangeuse de cations fort (type PUROLITE^{®} C120, C150), il est possible d'éliminer plus de 85% des cations tout en garantissant un rendement en méthionine > 95% par dimensionnement correct du volume de résine. En effet, la méthionine étant amphotère, elle va se fixer également sur la résine mais l'affinité des cations pour la résine est plus importante que celle de la méthionine : il y a alors déplacement de la méthionine par les cations.

Par le même mécanisme d'affinité, le perméat traité sur résine échangeuse d'anions (type Rohm & Haas FPA91, Bayer 4228,...) voie sa teneur en anions diminuer de plus de 95% avec un rendement en méthionine > 80%.

Après traitement de déminéralisation, la solution a la composition suivante :

**Tableau 3**

| **Composés** | **Teneur (%/sec)** |
|---|---|
| L-méthionine (MET) | 79,38 |
| N-Acétyl-Méthionne (NAM) | 1,49 |
| Isoleucine (ISO) | 0,54 |
| AA hors MET, NAM et ISO | 4,36 |
| Sucres totaux | 0,77 |
| Lipides | 1,18 |
| Acide succinique | 1,10 |
| Acide citrique | 0,16 |
| Thiosulfate | 0,23 |
| Sulfate | 0,12 |
| Phosphate | 0,05 |
| Chlorure | 0,10 |
| NH4 | 0,25 |
| Na | 0,08 |
| K | 0,11 |
| Mg | 0,10 |
| Ca | 0,02 |
| Fe | 0,01 |
| Autres | 9,55 |

Cette solution est ensuite concentrée afin de cristalliser la L-méthionine.

### D) Cristallisation

La solution déminéralisée est pré-concentrée par évaporation de l'eau à 50°C sur un évaporateur sous vide à film tombant de type WIEGAND^{®}. Le facteur de concentration est de l'ordre de 2 à 5 selon la concentration initiale en L-méthionine.

Il est ici égal à 3 pour se rapprocher de la sursaturation à 50°C (80g/L).

La solution pré-concentrée est alors transféré dans un évapocristallisoir à circulation forcée pour y être concentrée davantage et cristalliser sous vide (50mBar) à environ 35°C. Le facteur de concentration appliqué dans cet évapocristallisoir est d'environ 3, de façon à atteindre 240g/L.

Après séparation sur filtre presse CHOQUENET^{®} et lavage avec un volume d'eau déminéralisée par volume de gâteau, les cristaux sont séchés sur un lit fluidisé à 45°C (type AEROMATIC^{®}).

Dans ces conditions le rendement de récupération de L-méthionine est > 80% pour une pureté > 85%/sec.

La composition de la L-méthionine brute solide est présentée dans le tableau suivant :

**Tableau 4**

| **g / 100g de résidu sec** | **Composition brute solide** |
|---|---|
| L-Méthionine (L-MET) | 89,49 |
| N-acétyl Méthionine (NAM) | 0,13 |
| Isoleucine (ISO) | 2,41 |
| Dérivé d'AA inconnu | 0,50 |
| Valine | 0,69 |
| Phénylalanine | 0,44 |
| Leucine | 0,20 |
| Acide aspartique | 0,09 |
| Thréonine | 0,19 |
| Alanine | 0,15 |
| Lysine | 0,05 |
| Arginine | 0,04 |
| Cystine | 0,02 |
| Glutamine | 0,13 |
| Tyrosine | 0,44 |
| Acide glutamique | 0,19 |
| Glycine | 0,18 |
| Citrulline | 0,61 |
| Total AA hors L-MET, NAM et ISO | 2,37 |
| Sucres totaux | 0,31 |
| Lipides | < 1 |
| Acides organiques | < 1 |
| Thiosulfates | 0,02 |
| Sulfates | 0,02 |
| Phosphates | 0,05 |
| Chlorures | 0,01 |
| Ammonium | 0,04 |
| Sodium | 0,01 |
| Potassium | 0,03 |
| Magnésium | < 0,01 |
| Calcium | < 0,01 |
| Autres | 3,95 |

La liqueur mère contient encore près de 40%/sec de méthionine. Pour optimiser le rendement global, il est possible de recycler la liqueur mère en amont du Process, en totalité ou en partie, sous forme liquide ou après un second jet de cristallisation, avant ou après un traitement approprié.

La méthionine brute solide obtenue à partir du procédé décrit dans l'exemple selon l'invention, a une stabilité hygroscopique comparable à celui de la méthionine chimique.

### Exemple 2 : Alimentation des animaux

La méthionine brute obtenue précédemment sous forme solide est ajoutée à un aliment de base pour poulets de chair.

L'aliment de base a la composition suivante :

**Tableau 5**

| | % |
|---|---|
| Blé | 61.93 |
| Graine de soja cuite | 16.20 |
| Huile de palme | 0.50 |
| Tourteaux de soja | 18.40 |
| Phosphate bicalcique | 0.94 |
| Carbonate farine | 0.86 |
| Sel | 0.24 |
| Bicarbonate de soude | 0.20 |
| Lysine 50% liquide | 0.20 |
| Chlor. Choline 75% | 0.06 |
| Prem. Thréonine 25 | 0.03 |
| Prem. Phytase | 0.04 |
| Premix Volaille | 0.40 |

Cet aliment est supplémenté en méthionine brute pour obtenir des compositions comprenant différentes teneurs en méthionine brute, à 0,05%, 0,1%, 0,15%, 0,2% et 0,25% de méthionine ajoutée (correspondant à respectivement environ 0,063%, 0,125%, 0,188%, 0,250% et 0,313% de méthionine brute solide ajoutée à l'aliment).

Les animaux sont répartis en groupes et nourris avec l'aliment de base comme contrôle, ou avec un aliment supplémenté en méthionine brute selon l'invention, puis pesés à 1, 14 et 28 jours.

On constate une nette amélioration des performances pour les aliments supplémentés avec la méthionine selon l'invention. Un optimum d'efficacité est identifié vers environ 0,4% de méthionine dans l'aliment final, soit de 0,1 à 0,15% de méthionine ajoutée (l'aliment de base à une teneur en méthionine de l'ordre de 0,29%).

En effectuant la même expérience avec de la DL-méthionine d'origine chimique, on observe un optimum vers environ 0,48% de méthionine dans l'aliment final, soit environ 0,19% de méthionine ajoutée.

La méthionine brute selon l'invention semble donc mieux utilisée par l'animal que la DL-méthionine d'origine chimique.

## Revendications

1. Additif alimentaire pour l'alimentation animale comprenant, voire consistant en, une composition de méthionine issue d'un procédé de fermentation comprenant :
- de 60 à 95%, notamment de 70 à 95%, en poids de méthionine,
- de 0,05 à 2,5%, notamment de 0,1 à 2%, en poids de N-acétyl méthionine, et
- de 0,05 à 3,5%, notamment de 0,2 à 3%, en poids d'isoleucine.

2. Additif alimentaire selon la revendication 1, **caractérisée en ce que** la composition de méthionine comprend en outre :
- de 1,0 à 6,0% en poids, notamment de 2,0 à 5,0% en poids, d'acides aminés autres que méthionine, N-acétyl méthionine et isoleucine
- de 0,1 à 2% en poids, notamment de 0,2 à 1,5% en poids, de sucres totaux et
- de 0,05 à 2,5% en poids, notamment de 0,1 à 2% en poids, de lipides.

3. Additif alimentaire selon la revendication 1 ou 2, **caractérisée en ce qu'**il se présente sous forme liquide.

4. Additif alimentaire selon l'une quelconque des revendications 1 à 3, **caractérisée en ce qu'**il comprend, voire consiste en, une composition comprenant :
- de 60 à 80% en poids, notamment de 65 à 75 % en poids, de méthionine,
- de 0,5 à 2,0 % en poids, notamment de 0,75 à 1,75% en poids, de N-acétyl méthionine, et
- de 0,20 à 1,5% en poids, notamment de 0,3 à 1,0%, en poids, d'isoleucine.

5. Additif alimentaire selon l'une quelconque des revendications 1 à 4, **caractérisée en ce qu'**il comprend, voire consiste en, une composition comprenant :
- de 2 à 6% en poids, notamment de 3 à 5% en poids, d'acides aminés autres que méthionine, N-acétyl méthionine et isoleucine
- de 0,5 à 2% en poids, notamment de 0,75 à 1,5% en poids, de sucres totaux et
- de 0,05 à 2,5% en poids, notamment de 0,75 à 1,5% en poids, de lipides.

6. Additif alimentaire l'une quelconque des revendications 1 à 3, **caractérisée en ce qu'**il comprend, voire consiste en, une composition comprenant :
- de 70 à 90% en poids, notamment de 75 à 85% en poids, de méthionine,
- de 0,05 à 2,5% en poids, notamment de 0,075 à 2,0% en poids, de N-acétyl méthionine, et
- de 0,10 à 1,0% en poids, notamment de 0,3 à 0,75%, en poids d'isoleucine.

7. Additif alimentaire l'une quelconque des revendications 1 à 3 et 6, **caractérisée en ce qu'**il comprend, voire consiste en, une composition comprenant :
- de 2 à 6% en poids, notamment de 3 à 5% en poids, d'acides aminés autres que méthionine, N-acétyl méthionine et isoleucine
- de 0,25 à 2% en poids, notamment de 0,5 à 1,5% en poids, de sucres totaux et
- de 0,05 à 2,5% en poids, notamment de 0,075 à 2,0% en poids, de lipides.

8. Additif alimentaire selon la revendication 1 ou 2, **caractérisée en ce qu'**il se présente sous forme solide.

9. Additif alimentaire selon l'une quelconque des revendications 1, 2 et 8, **caractérisée en ce qu'**il comprend, voire consiste en, une composition comprenant :
- de 80 à 95% en poids, notamment de 85 à 95% en poids, de méthionine,
- de 0,05 à 0,5% en poids, notamment de 0,05 à 0,3% en poids, de N-acétyl méthionine, et
- de 0,05 à 5,0% en poids, notamment de 0,1 à 3,0%, en poids d'isoleucine.

10. Additif alimentaire selon l'une quelconque des revendications 1, 2, 8 et 9, **caractérisée en ce qu'**il comprend, voire consiste en, une composition comprenant :
- de 1,0 à 5,0% en poids, notamment de 1,5 à 4,0% en poids, d'acides aminés autres que méthionine, N-acétyl méthionine et isoleucine
- de 0,1 à 1,5% en poids, notamment de 0,2 à 1,0% en poids, de sucres totaux et
- moins de 0,3% en poids de lipides.

11. Procédé de préparation d'un additif alimentaire pour l'alimentation animale, notamment tel que défini selon l'une quelconque des revendications 1 à 10, **caractérisé en ce qu'**il comprend, voire consiste en, au moins l'étape suivante consistant, à partir d'un milieu de fermentation d'un procédé de préparation de méthionine dans lequel on cultive un microorganisme modifié pour produire de la méthionine sur un milieu de culture approprié comprenant une source de carbone, à :
**A)** clarifier le milieu de fermentation et éliminer les impuretés organiques insolubles et solubles dudit milieu de fermentation, notamment pour l'obtention d'une composition brute liquide comprenant :
- de 60 à 80% en poids, notamment de 65 à 75% en poids, de méthionine,
- de 0,5 à 2,0% en poids, notamment de 0,75 à 1,75% en poids, de N-acétyl méthionine, et
- de 0,20 à 1,5% en poids, notamment de 0,3 à 1,0%, en poids, d'isoleucine, et
**E)** récupérer l'additif alimentaire.

12. Procédé de préparation d'un additif alimentaire selon la revendication 11, **caractérisé en ce que** la composition brute liquide comprend en outre :
- de 2 à 6% en poids, notamment de 3 à 5% en poids, d'acides aminés autres que méthionine, N-acétyl méthionine et isoleucine
- de 0,1 à 2% en poids, notamment de 0,75 à 1,5% en poids, de sucres totaux et
- de 0,05 à 2,5% en poids, notamment de 0,75 à 1,5% en poids, de lipides.

13. Procédé de préparation d'un additif alimentaire selon la revendication 11 ou 12, **caractérisé en ce qu'**il comprend en outre l'étape consistant à :
**B)** déminéraliser la solution brute liquide afin d'éliminer les cations et anions dudit milieu de fermentation, notamment pour l'obtention d'une composition brute liquide déminéralisée comprenant :
- de 70 à 90% en poids, notamment de 75 à 85% en poids, de méthionine,
- de 0,05 à 2,5% en poids, notamment de 0,075 à 2,0% en poids, de N-acétyl méthionine, et
- de 0,10 à 1,0% en poids, notamment de 0,3 à 0,75%, en poids d'isoleucine.

14. Procédé de préparation d'un additif alimentaire selon la revendication 13, **caractérisé en ce que** la composition brute liquide déminéralisée comprend en outre :
- de 2 à 6% en poids, notamment de 3 à 5% en poids, d'acides aminés autres que méthionine, N-acétyl méthionine et isoleucine
- de 0,25 à 2% en poids, notamment de 0,5 à 1,5% en poids, de sucres totaux et
- de 0,05 à 2,5% en poids, notamment de 0,075 à 2,0% en poids, de lipides.

15. Procédé de préparation d'un additif alimentaire selon l'une quelconque des revendications 11 à 14, **caractérisé en ce que** l'additif alimentaire est liquide, et notamment ledit procédé ne comprend pas d'étape de cristallisation.

16. Procédé de préparation d'un additif alimentaire selon l'une quelconque des revendications 11 à 15, **caractérisé en ce qu'**il comprend en outre l'étape consistant à **C)** cristalliser de la méthionine brute solide à partir de la composition brute liquide, éventuellement déminéralisée.

17. Procédé de préparation d'un additif alimentaire selon la revendication 16, **caractérisé en ce qu'**il comprend en outre l'étape consistant à
**D)** séparer, éventuellement laver, et éventuellement broyer des cristaux de méthionine pour l'obtention d'une composition brute solide.

18. Procédé de préparation d'un additif alimentaire selon la revendication 16 ou 17, **caractérisé en ce que** la composition brute solide comprend
- de 80 à 95% en poids, notamment de 85 à 95% en poids, de méthionine,
- de 0,05 à 0,5% en poids, notamment de 0,05 à 0,3% en poids, de N-acétyl méthionine, et
- de 0,05 à 5,0% en poids, notamment de 0,1 à 3,0%, en poids d'isoleucine.

19. Procédé de préparation d'un additif alimentaire selon la revendication 18, **caractérisé en ce que** la composition méthionine brute solide ou la composition brute solide comprend en outre :
- de 1,0 à 5,0% en poids, notamment de 1,5 à 4,0% en poids, d'acides aminés autres que méthionine, N-acétyl méthionine et isoleucine,
- de 0,1 à 1,5% en poids, notamment de 0,2 à 1,0% en poids, de sucres totaux et
- moins de 0,3% en poids de lipides.

## Patentansprüche

1. Futterzusatz für Futtermittel, umfassend eine oder bestehend aus einer Methionin-Zusammensetzung, die aus einem Fermentationsverfahren stammt und umfasst:
- 60 bis 95 Gew.-%, bevorzugt 70 bis 95 Gew.-%, Methionin,
- 0,05 bis 2,5 Gew.-%, bevorzugt 0,1 bis 2 Gew.-%, N-Acetylmethionin, und
- 0,05 bis 3,5 Gew.-%, bevorzugt 0,2 bis 3 Gew.-%, Isoleucin.

2. Futterzusatz gemäß Anspruch 1, **dadurch gekennzeichnet, dass** die Methionin-Zusammensetzung außerdem umfasst:
- 1,0 bis 6,0 Gew.-%, bevorzugt 2,0 bis 5,0 Gew.-%, weitere Aminosäuren außer Methionin, N-Acetylmethionin und Isoleucin,
- 0,1 bis 2 Gew.-%, bevorzugt 0,2 bis 1,5 Gew.-%, Gesamtzucker, und
- 0,05 bis 2,5 Gew.-%, bevorzugt 0,1 bis 2 Gew.-%, Lipide.

3. Futterzusatz gemäß Anspruch 1 oder 2, **dadurch gekennzeichnet, dass** er in flüssiger Form vorliegt.

4. Futterzusatz gemäß einem der Ansprüche 1 bis 3, **dadurch gekennzeichnet, dass** er eine Zusammensetzung umfasst oder aus dieser besteht, umfassend:
- 60 bis 80 Gew.-%, bevorzugt 65 bis 75 Gew.-%, Methionin,
- 0,5 bis 2,0 Gew.-%, bevorzugt 0,75 bis 1,75 Gew.-%, N-Acetxlmethionin, und
- 0,20 bis 1,5 Gew.-%, bevorzugt 0,3 bis 1,0 Gew.-%, Isoleucin.

5. Futterzusatz gemäß einem der Ansprüche 1 bis 4, **dadurch gekennzeichnet, dass** er eine Zusammensetzung umfasst oder aus dieser besteht, umfassend:
- 2 bis 6 Gew.-%, bevorzugt 3 bis 5 Gew.-%, weitere Aminosäuren außer Methionin, N-Acetylmethionin und Isoleucin,
- 0,5 bis 2 Gew.-%, bevorzugt 0,75 bis 1,5 Gew.-%, Gesamtzucker, und
- 0,05 bis 2,5 Gew.-%, bevorzugt 0,75 bis 1,5 Gew.-%, Lipide.

6. Futterzusatz gemäß einem der Ansprüche 1 bis 3, **dadurch gekennzeichnet, dass** er eine Zusammensetzung umfasst oder aus dieser besteht, umfassend:
- 70 bis 90 Gew.-%, bevorzugt 75 bis 85 Gew.-%, Methionin,
- 0,05 bis 2,5 Gew.-%, bevorzugt 0,075 bis 2,0 Gew.-%, N-Acetylmethionin, und
- 0,10 bis 1,0 Gew.-%, bevorzugt 0,3 bis 0,75 Gew.-%, Isoleucin.

7. Futterzusatz gemäß einem der Ansprüche 1 bis 3 und 6, **dadurch gekennzeichnet, dass** er eine Zusammensetzung umfasst oder aus dieser besteht, umfassend:
- 2 bis 6 Gew.-%, bevorzugt 3 bis 5 Gew.-%, weitere Aminosäuren außer Methionin, N-Acetylmethionin und Isoleucin,
- 0,25 bis 2 Gew.-%, bevorzugt 0,5 bis 1,5 Gew.-%, Gesamtzucker, und
- 0,05 bis 2,5 Gew.-%, bevorzugt 0,075 bis 2,0 Gew.-%, Lipide.

8. Futterzusatz gemäß Anspruch 1 oder 2, **dadurch gekennzeichnet, dass** er in fester Form vorliegt.

9. Futterzusatz gemäß einem der Ansprüche 1, 2 und 8, **dadurch gekennzeichnet, dass** er eine Zusammensetzung umfasst oder aus dieser besteht, umfassend:
- 80 bis 95 Gew.-%, bevorzugt 85 bis 95 Gew.-%, Methionin,
- 0,05 bis 0,5 Gew.-%, bevorzugt 0,05 bis 0,3 Gew.-%, N-Acetylmethionin, und
- 0,05 bis 5,0 Gew.-%, bevorzugt 0,1 bis 3,0 Gew.-%, Isoleucin.

10. Futterzusatz gemäß einem der Ansprüche 1, 2, 8 und 9, **dadurch gekennzeichnet, dass** er eine Zusammensetzung umfasst oder aus dieser besteht, umfassend:
- 1,0 bis 5,0 Gew.-%, bevorzugt 1,5 bis 4,0 Gew.-%, weitere Aminosäuren außer Methionin, N-Acetylmethionin und Isoleucin,
- 0,1 bis 1,5 Gew.-%, bevorzugt 0,2 bis 1,0 Gew.-%, Gesamtzucker, und
- weniger als 0,3 Gew.-% Lipide.

11. Verfahren zur Herstellung eines Futterzusatzes für Futtermittel, insbesondere wie er nach einem der Ansprüche 1 bis 10 definiert ist, **dadurch gekennzeichnet, dass** das Verfahren wenigstens den folgenden Schritt umfasst oder aus diesem besteht, bestehend aus, ausgehend von einem Fermentationsmedium eines Verfahrens zur Methionin-Herstellung, in dem man einen veränderten Mikroorganismus kultiviert, um Methionin in einem eine Kohlenstoffquelle umfassenden geeigneten Kulturmedium zu produzieren:
**A)** Klärung des Fermentationsmediums und Entfernung löslicher und unlöslicher organischer Verunreinigungen aus besagtem Fermentationsmedium, insbesondere um eine flüssige Rohzusammensetzung zu erhalten, umfassend:
- 60 bis 80 Gew.-%, bevorzugt 65 bis 75 Gew.-%, Methionin,
- 0,5 bis 2,0 Gew.-%, bevorzugt 0,75 bis 1,75 Gew.-%, N-Acetylmethionin, und
- 0,20 bis 1,5 Gew.-%, bevorzugt 0,3 bis 1,0 Gew.-%, Isoleucin, und
**E)** Wiedergewinnung des Futterzusatzes.

12. Verfahren zur Herstellung eines Futterzusatzes gemäß Anspruch 11, **dadurch gekennzeichnet, dass** die flüssige Rohzusammensetzung außerdem umfasst:
- 2 bis 6 Gew.-%, bevorzugt 3 bis 5 Gew.-%, weitere Aminosäuren außer Methionin, N-Acetylmethionin und Isoleucin,
- 0,1 bis 2 Gew.-%, bevorzugt 0,75 bis 1,5 Gew.-%, Gesamtzucker, und
- 0,05 bis 2,5 Gew.-%, bevorzugt 0,75 bis 1,5 Gew.-%, Lipide.

13. Verfahren zur Herstellung eines Futterzusatzes gemäß Anspruch 11 oder 12, **dadurch gekennzeichnet, dass** es den Schritt umfasst, bestehend aus:
**B)** Demineralisierung der flüssigen Rohlösung, um Kationen und Anionen aus besagtem Fermentationsmedium zu entfernen, insbesondere um eine demineralisierte flüssige Rohzusammensetzung zu erhalten, umfassend:
- 70 bis 90 Gew.-%, bevorzugt 75 bis 85 Gew.-%, Methionin,
- 0,05 bis 2,5 Gew.-%, bevorzugt 0,075 bis 2,0 Gew.-%, N-Acetylmethionin, und
- 0,10 bis 1,0 Gew.-%, bevorzugt 0,3 bis 0,75 Gew.-%, Isoleucin.

14. Verfahren zur Herstellung eines Futterzusatzes gemäß Anspruch 13, **dadurch gekennzeichnet, dass** die demineralisierte flüssige Rohzusammensetzung außerdem umfasst:
- 2 bis 6 Gew.-%, bevorzugt 3 bis 5 Gew.-%, weitere Aminosäuren außer Methionin, N-Acetylmethionin und Isoleucin,
- 0,25 bis 2 Gew.-%, bevorzugt 0,5 bis 1,5 Gew.-%, Gesamtzucker, und
- 0,05 bis 2,5 Gew.-%, bevorzugt 0,075 bis 2,0 Gew.-%, Lipide.

15. Verfahren zur Herstellung eines Futterzusatzes gemäß einem der Ansprüche 11 bis 14, **dadurch gekennzeichnet, dass** der Futterzusatz flüssig ist und insbesondere besagtes Verfahren

16. Verfahren zur Herstellung eines Futterzusatzes gemäß einem der Ansprüche 11 bis 15, **dadurch gekennzeichnet, dass** es außerdem den Schritt umfasst, bestehend aus:
**C)** Kristallisation des festen Rohmethionins aus der, eventuell demineralisierten, flüssigen Rohzusammensetzung.

17. Verfahren zur Herstellung eines Futterzusatzes gemäß Anspruch 16, **dadurch gekennzeichnet, dass** es außerdem den Schritt umfasst, bestehend aus:
**D)** Trennen, eventuell Waschen und eventuell Zerkleinern der Methionin-Kristalle, um eine feste Rohzusammensetzung zu erhalten.

18. Verfahren zur Herstellung eines Futterzusatzes gemäß Anspruch 16 oder 17, **dadurch gekennzeichnet, dass** die feste Rohzusammensetzung umfasst:
- 80 bis 95 Gew.-%, bevorzugt 85 bis 95 Gew.-%, Methionin,
- 0,05 bis 0,5 Gew.-%, bevorzugt 0,05 bis 0,3 Gew.-%, N-Acetylmethionin, und
- 0,05 bis 5,0 Gew.-%, bevorzugt 0,1 bis 3,0 Gew.-%, Isoleucin.

19. Verfahren zur Herstellung eines Futterzusatzes gemäß Anspruch 18, **dadurch gekennzeichnet, dass** die feste Methionin-Rohzusammensetzung oder die feste Rohzusammensetzung außerdem umfasst:
- 1,0 bis 5,0 Gew.-%, bevorzugt 1,5 bis 4,0 Gew.-%, weitere Aminosäuren außer Methionin, N-Acetylmethionin und Isoleucin,
- 0,1 bis 1,5 Gew.-%, bevorzugt 0,2 bis 1,0 Gew.-% Gesamtzucker, und
- weniger als 0,3 Gew.-% Lipide.

## Claims

1. A feed additive for feeding animals, comprising, or consisting of, a methionine composition resulting from a fermentation process, comprising:
- from 60 to 95%, especially from 70 to 95%, by weight of methionine,
- from 0.05 to 2.5%, especially from 0.1 to 2%, by weight of N-acetylmethionine, and
- from 0.05 to 3.5%, especially from 0.2 to 3%, by weight of isoleucine.

2. The feed additive according to claim 1, **characterized in that** the methionine composition further comprises:
- from 1.0 to 6.0% by weight, especially from 2.0 to 5.0% by weight, of amino acids other than methionine, N-acetylmethionine and isoleucine,
- from 0.1 to 2% by weight, especially from 0.2 to 1.5% by weight, of total sugars, and
- from 0.05 to 2.5% by weight, especially from 0.1 to 2% by weight, of lipids.

3. The feed additive according to claim 1 or 2, **characterized in that** it is in liquid form.

4. The feed additive according to any one of claims 1 to 3, **characterized in that** it comprises, or consists of, a composition comprising:
- from 60 to 80% by weight, especially from 65 to 75% by weight, of methionine,
- from 0.5 to 2.0% by weight, especially from 0.75 to 1.75% by weight, of N-acetylmethionine, and
- from 0.20 to 1.5% by weight, especially from 0.3 to 1.0% by weight, of isoleucine.

5. The feed additive according to any one of claims 1 to 4, **characterized in that** it comprises, or consists of, a composition comprising:
- from 2 to 6% by weight, especially from 3 to 5% by weight, of amino acids other than methionine, N-acetylmethionine and isoleucine,
- from 0.5 to 2% by weight, especially from 0.75 to 1.5% by weight, of total sugars, and
- from 0.05 to 2.5% by weight, especially from 0.75 to 1.5% by weight, of lipids.

6. The feed additive according to any one of claims 1 to 3, **characterized in that** it comprises, or consists of, a composition comprising:
- from 70 to 90% by weight, especially from 75 to 85% by weight, of methionine,
- from 0.05 to 2.5% by weight, especially from 0.075 to 2.0% by weight, of N-acetylmethionine, and
- from 0.10 to 1.0% by weight, especially from 0.3 to 0.75% by weight, of isoleucine.

7. The feed additive according to any one of claims 1 to 3 and 6, **characterized in that** it comprises, or consists of, a composition comprising:
- from 2 to 6% by weight, especially from 3 to 5% by weight, of amino acids other than methionine, N-acetylmethionine and isoleucine,
- from 0.25 to 2% by weight, especially from 0.5 to 1.5% by weight, of total sugars, and
- from 0.05 to 2.5% by weight, especially from 0.075 to 2.0% by weight, of lipids.

8. The feed additive according to claim 1 or 2, **characterized in that** it is in solid form.

9. The feed additive as claimed in any one of claims 1, 2 and 8, **characterized in that** it comprises, or consists of, a composition comprising:
- from 80 to 95% by weight, especially from 85 to 95% by weight, of methionine,
- from 0.05 to 0.5% by weight, especially from 0.05 to 0.3% by weight, of N-acetylmethionine, and
- from 0.05 to 5.0% by weight, especially from 0.1 to 3.0% by weight, of isoleucine.

10. The feed composition according to any one of claims 1, 2, 8 and 9, **characterized in that** it comprises, or consists of, a composition comprising:
- from 1.0 to 5.0% by weight, especially from 1.5 to 4.0% by weight, of amino acids other than methionine, N-acetylmethionine and isoleucine,
- from 0.1 to 1.5% by weight, especially from 0.2 to 1.0% by weight, of total sugars, and
- less than 0.3% by weight of lipids.

11. A process for preparing a feed additive for feeding animals, especially as defined according to any one of claims 1 to 10, **characterized in that** it comprises, or consists of, at least the following step which, using a fermentation medium from a methionine preparation process in which a microorganism that has been modified to produce methionine is cultured on a suitable culture medium comprising a carbon source, consists in:
**A)** clarifying the fermentation medium and removing the insoluble and soluble organic impurities from said fermentation medium, especially in order to obtain a liquid crude composition comprising:
- from 60 to 80% by weight, especially from 65 to 75% by weight, of methionine,
- from 0.5 to 2.0% by weight, especially from 0.75 to 1.75% by weight, of N-acetylmethionine, and
- from 0.20 to 1.5% by weight, especially from 0.3 to 1.0% by weight, of isoleucine, and
**E)** recovering the feed additive.

12. The process for preparing a feed additive according to claim 11, **characterized in that** the liquid crude composition further comprises:
- from 2 to 6% by weight, especially from 3 to 5% by weight, of amino acids other than methionine, N-acetylmethionine and isoleucine,
- from 0.1 to 2% by weight, especially from 0.75 to 1.5% by weight, of total sugars, and
- from 0.05 to 2.5% by weight, especially from 0.75 to 1.5% by weight, of lipids.

13. The process for preparing a feed additive according to claim 11 or 12, **characterized in that** it further comprises the step of:
**B)** demineralizing the liquid crude solution in order to remove the cations and anions from said fermentation medium, especially in order to obtain a demineralized liquid crude composition comprising:
- from 70 to 90% by weight, especially from 75 to 85% by weight, of methionine,
- from 0.05 to 2.5% by weight, especially from 0.075 to 2.0% by weight, of N-acetylmethionine, and
- from 0.10 to 1.0% by weight, especially from 0.3 to 0.75% by weight, of isoleucine.

14. The process for preparing a feed additive according to claim 13, **characterized in that** the demineralized liquid crude composition further comprises:
- from 2 to 6% by weight, especially from 3 to 5% by weight, of amino acids other than methionine, N-acetylmethionine and isoleucine,
- from 0.25 to 2% by weight, especially from 0.5 to 1.5% by weight, of total sugars, and
- from 0.05 to 2.5% by weight, especially from 0.075 to 2.0% by weight, of lipids.

15. The process for preparing a feed additive according to any one of claims 11 to 14, **characterized in that** the feed additive is liquid, and especially said process does not comprise a crystallization step.

16. The process for preparing a feed additive according to any one of claims 11 to 15, **characterized in that** it further comprises the step of:
**C)** crystallizing solid crude methionine from the liquid crude composition, optionally demineralized.

17. The process for preparing a feed additive according to claim 16, **characterized in that** it further comprises the step of:
**D)** separating, optionally washing, and optionally milling methionine crystals in order to obtain a solid crude composition.

18. The process for preparing a feed additive according to claim 16 or 17, **characterized in that** the solid crude composition comprises:
- from 80 to 95% by weight, especially from 85 to 95% by weight, of methionine,
- from 0.05 to 0.5% by weight, especially from 0.05 to 0.3% by weight, of N-acetylmethionine, and
- from 0.05 to 5.0% by weight, especially from 0.1 to 3.0% by weight, of isoleucine.

19. The process for preparing a feed additive according to claim 18, **characterized in that** the solid crude methionine composition or the solid crude composition further comprises:
- from 1.0 to 5.0% by weight, especially from 1.5 to 4.0% by weight, of amino acids other than methionine, N-acetylmethionine and isoleucine,
- from 0.1 to 1.5% by weight, especially from 0.2 to 1.0% by weight, of total sugars, and
- less than 0.3% by weight of lipids.
